# EUROPEAN PATENT APPLICATION

(11) **EP 4 339 961 A1**
(43) Date of publication of application: **20.03.2024**
(21) Application number: 22195531.3
(22) Date of filing: 14.09.2022
(51) Int. Cl.: G16H 15/00

(54) **METHODS AND SYSTEMS FOR PROVIDING A TEMPLATE DATA STRUCTURE FOR A MEDICAL REPORT**

(71) Applicant: Siemens Healthineers AG, 91301 Forchheim (DE)
(72) Inventor: Huber, Martin, 91080 Uttenreuth (DE); Kohl, Gerhard, 91077 Neunkirchen am Brand (DE); Kohle, Sven, 91056 Erlangen (DE); Speier, Christoph, 91052 Erlangen (DE); Tietjen, Christian, 90763 Fürth (DE)
(74) Representative: Siemens Healthineers Patent Attorneys

(57) **Abstract**

Systems and computer-implemented methods are provided for identifying a template data structure (TDS) for a medical report (MER) based on a medical image data set (MIDS). Specifically, the following steps are disclosed:
- receiving (S10) a medical image data set (MIDS) of a patient,
- generating (S20) a representation (R) of the medical image data set (MIDS) for displaying to a user via a user interface (10),
- providing (S30) the representation (R) for displaying to the user via the user interface (10),
- receiving (S40) a user input (INP) via the user interface (10) directed to a medical finding (MF) visible in the representation (R),
- predicting (S50) a finding type (FT) of the medical finding (MF) based on the user input (INP),
- performing (S60) a lookup operation in a database (RD) to identify a template data structure (TDS) for a medical report (MER) corresponding to the predicted finding type (FT), and
- providing (S70) the identified template data structure (TDS) .

## Description

The invention concerns systems and methods in the field of automatically or semi-automatically generating structured medical reports. Specifically, the invention concerns systems and methods for providing one or more template data structures which may serve as building blocks for a medical report.

When generating a radiological or pathological report, the reviewing radiologist or pathologist summarizes her or his observations made when reviewing medical images. The report can be a free-text report. Then, its structure, elements, style, wording, and layout may differ from physician to physician. They are not straight-forwardly machine-readable, not standardized, and not analyzable. Moreover, they are prone to artefacts, and they might be unclear, difficult to interpret, or even incomplete.

To overcome the drawbacks of free-text reports, so-called structured reports were introduced. These are based on structured, machine-readable reporting templates that can be combined and progressively filled by the user to provide the final medical report. Ideally, a structured report is machine-readable, has a fixed structure and contains standardized elements, wording, and layout. In addition, pre-generated report templates or modules as building blocks for the medical report can be used. These may provide case-specific structure and include recommended reporting steps.

While structured reporting can improve the quality and the interoperability of medical reports, this may also increase the burden for the individual user upon preparing the report. Rather than more or less freely dictating the medical findings, the user has to select the correct templates or building blocks and fill these in a predetermined manner. What is more, there usually is a huge variety of different templates a radiologist has to choose from. Finding the correct template is often crucial for the entire process, as a wrong template may set the reporting and therewith the entire diagnosis on the wrong track. In turn, selecting the correct templates is a difficult task as this may depend on various factors such as the diagnostic task, the circumstances of the case, or the data available. Additionally, there rarely is a singular template for a medical report. Rather, different modules or sub-templates have to combined to arrive at a comprehensive medical report.

Typically, radiologists must perform several tasks in parallel when evaluating medical image data and preparing a report. First and foremost, they must analyze the medical images and summarize their observations and impressions in a radiological report. In addition, they must further take into account additional information about the patient. This information can, for example, come from images of different modalities or measurement protocols, as well as from information in the patient's health record, laboratory findings, previous images, etc. Thus, the types and number of the individual reporting tasks to be undertaken depends on the available interdisciplinary data, i.e., medical images and other available patient specific medical data.

To assist users in preparing structured reports, several concepts have been developed. For instance, the usage of checklists has been proposed. Based on these checklists, a user may be guided through the proposed reporting steps. Other approaches are based on electronic decision trees where the individual nodes are linked to different report templates. Thus, when moving along the decision tree, the correct templates can automatically be retrieved.

One issue with suchlike solutions is that medical reporting often is a highly dynamic task and both checklists and decision trees often prove to be too rigid to map real-world reporting workflows. This is because the types and number of the individual reporting tasks to be undertaken depends on the findings and the available data. Moreover, it often only becomes apparent during the process of reporting which data is actually used and which diagnostic steps have to be carried out. This is because new data and information are generated during this process, which may then indicate further steps. E.g., secondary findings may be made that require additional reporting tasks.

Against this background it is an object of embodiments of the present invention to provide systems and methods for providing template data structures for a medical report so as to better support a user in providing a structured medical report. In particular, it is an object of embodiments of the present invention to provide systems and methods allowing for the targeted retrieval of template data structures for a medical report based on the reporting actions performed by the user.

In the following, the technical solution according to the present invention is described with respect to the claimed systems as well as with respect to the claimed methods. Features, advantages, or alternative embodiments described herein can likewise be assigned to other claimed objects and vice versa. In other words, claims addressing the inventive method can be improved by features described or claimed with respect to the systems. In this case, e.g., functional features of the methods are embodied by objective units or elements of the systems.

The technical solution will be described both with regard to methods and systems for providing a template data structure for a medical report and also with regard to methods and systems for providing trained functions. Features and alternate forms of embodiments of data structures and/or functions for methods and systems for providing can be transferred to analogous data structures and/or functions for methods and systems for providing trained functions. Analogous data structures can, in particular, be identified by using the prefix "training". Furthermore, the trained functions used in methods and system for providing a template data structure for a medical report can, in particular, have been adjusted and/or trained and/or provided by methods and systems for adjustment of trained functions.

According to an aspect, a computer-implemented method for providing a template data structure for a medical report is provided. The method comprises a plurality of steps. A first step is directed to receive a medical image data set of a patient. Another step is directed to generate a representation of the medical image data set for displaying to a user via a user interface. Another step is directed to provide the representation for displaying to the user via the user interface. Another step is directed to receive a user input via the user interface directed to a medical finding visible in the representation. Another step is directed to predict a finding type of the medical finding based on the user input. Another step is directed to performing a lookup operation in a database to identify a template data structure for a medical report corresponding to the predicted finding type. Another step is directed to provide the identified template data structure.

The medical image data set may be a two-dimensional image. Further, the medical image data set may be a three-dimensional image. Further, the medical image may be a four-dimensional image, where there are three spatial and one time-like dimensions. Further, the medical image data set may comprise a plurality of individual medical images.

The medical image data set comprises image data, for example, in the form of a two- or three-dimensional array of pixels or voxels. Such arrays of pixels or voxels may be representative of color, intensity, absorption or other parameters as a function of two or three-dimensional position, and may, for example, be obtained by suitable processing of measurement signals obtained by a medical imaging modality or image scanning facility.

The medical image data set may be a radiology image data set depicting a body part of a patient. Accordingly, it may contain two or three-dimensional image data of the patient's body part. The medical image may be representative of an image volume or a cross-section through the image volume. The patient's body part may be comprised in the image volume.

A medical imaging modality corresponds to a system used to generate or produce medical image data. For example, a medical imaging modality may be a computed tomography system (CT system), a magnetic resonance system (MR system), an angiography (or C-arm X-ray) system, a positron-emission tomography system (PET system) or the like. Specifically, computed tomography is a widely used imaging method and makes use of "hard" X-rays produced and detected by a spatially rotating instrument. The resulting attenuation data (also referred to as raw data) is processed by a computed analytic software producing detailed images of the internal structure of the patient's body parts. The produced sets of images are called CT-scans which may constitute multiple series of sequential images to present the internal anatomical structures in cross sections perpendicular to the axis of the human body. Magnetic Resonance Imaging (MRI), to provide another example, is an advanced medical imaging technique which makes use of the effect magnetic field impacts on movements of protons. In MRI machines, the detectors are antennas and the signals are analyzed by a computer creating detailed images of the internal structures in any section of the human body.

Accordingly, the depicted body part of the patient in general will comprise a plurality of anatomies and/or organs. Taking a chest image as an example, the medical image may show lung tissue, the rib cage, lymph nodes and others.

A medical image data set may comprise a plurality of images or image slices. The slices respectively show a cross-sectional view of the image volume. The slices may comprise a two-dimensional array of pixels or voxels as image data. The arrangement of slices in the medical image data set may be determined by the imaging modality or by any post-processing scheme used. Further, slices may artificially be defined in the imaging volume spanned by the medical image data set. Optionally, this may happen as a function of the image data comprised in the medical image data set in order to optimally pre-process the medical image data set for the ensuing diagnostic workflow.

Further the medical image data set may be a two-dimensional pathology image data set, i.e., a so-called whole-slide image. A whole-slide image may image a tissue slice or slide of a patient. The tissue slice may be prepared from tissue samples taken from the patient. Further, the preparation of a tissue slice may comprise the staining of the tissue slice with a histopathological staining. The staining in this case can serve to highlight different structures in the tissue slice, such as, e.g., cell walls or cell nuclei, or to test a medical indication, such as, e.g., a cell proliferation level. To create the whole-slide image, the stained tissue slices are digitized or scanned. To this end, the tissue slices are scanned with a suitable digitizing station, such as, for example, a whole-slide scanner, which preferably scans the entire tissue slice mounted on an object carrier and converts it into a pixel image.

The medical image may be stored in a standard image format such as the Digital Imaging and Communications in Medicine (DICOM) format and in a memory or computer storage system such as a Picture Archiving and Communication System (PACS), a Radiology Information System (RIS), and the like. Whenever DICOM is mentioned herein, it shall be understood that this refers to the "Digital Imaging and Communications in Medicine" (DICOM) standard, for example according to the DICOM PS3.1 2020c standard (or any later or earlier version of said standard).

The representation may be a two-dimensional representation image rendered from the medical image data set for displaying to a user in a user interface. The representation may comprise a plurality of image pixels. In particular, the representation may be a two-dimensional rendering of the medical image. Two-dimensional renderings may, in general, rely on known rendering procedures, such as ray-casting, ray-tracing, texture-rendering or the like. According to some examples, the rendering may be such that already identified medical findings and/or any candidate medical findings are displayed in conjunction with the image data of the medical image.

The user input may be any input directed to designate a medical finding. The user input may comprise a voice command or any other, in particular, physical input into a user interface, in particular, a graphical user interface. For instance, the user may use input devices like a computer-mouse, a trackball device, a smart pen, a touch pad, a touch sensitive display, etc. Further, the user input may be captured by eye tracking or by tracking gestures. The user input may, in particular, comprise designating a medical finding directly in the representation, e.g., by clicking, drawing contours, or invoking a measurement tool in a specific location in the representation. The user input may be such that it fully lines out the medical finding or such that it indicates only parts or even only a point in the representation which is then automatically related to a possible medical finding.

According to some implementations the user input may comprise a plurality of individual user interactions with a user interface (such as user inputs with regard to the representation, displaying settings, general settings, measurements, etc.).

According to some implementations, the user input may also comprise activating a computer-aided detection algorithm configured to detect medical findings in medical image data and, optionally, navigating to one of the findings detected by the computer aided detection algorithm.

Computer aided detection algorithms may generally be configured to detect candidate medical findings in medical images. For instance, the findings detection algorithms may have two stages: the detection stage for detecting potentially relevant patterns in image data and the classification stage for classifying the potentially relevant patterns either as candidate medical findings or as false positives to be discarded. In principle, a plethora of functionalities and methods is known for such computer aided detection and classification of candidate medical findings - all of which may be implemented in the findings detection algorithms. For instance, reference is made to US 2009 / 0 092 300 A1, US 2009 / 0 067 693 A1, and US 2016 / 0 321 427 A1, the contents of which are incorporated herein in their entirety by reference.

Each medical finding may relate to corresponding image data in the medical image. A medical finding may indicate a certain condition or pathology of the patient. The condition or pathology may be relevant for the diagnosis of the patient.

A medical finding may relate to an anatomical structure that differentiates the patient from other patients. Medical findings may be located within different organs of the patient (e.g., within the lung of a patient, or within the liver of a patient) or in between the organs of the patient. In particular, a medical finding may also relate to a foreign body.

In particular, a medical finding may relate to a neoplasm (also denoted as "tumor"), in particular, a benign neoplasm, an in-situ neoplasm, a malignant neoplasm and/or a neoplasm of uncertain/unknown behavior. In particular, a medical finding may relate to a nodule, in particular, a lung nodule. In particular, a medical finding may relate to a lesion, in particular, a lung lesion.

Medical findings may be classified according to their type or category. This type or category is called "finding type". A finding type may specify the general nature of medical finding. Further, the finding type may specify the anatomy or organ in which a medical finding has been found. According to some implementations, the finding type may also be conceived as a label of the medical finding. For instance, finding types may be lung nodule, liver nodule, cyst, rib fracture, undefined lesion, etc.

A template data structure may be a building block or module or sub-template on the basis of which a structured medical report may be generated. A medical report may be generated based on at least one, in general a plurality of template data structures. Specifically, a plurality of different template data structures may be combined to form a medical report.

Each template data structure may be specific to a certain finding type. For instance, a certain template data structure may be associated to lung nodules, while another template data structure is associated to liver nodules.

Each template data structure may specify one or more data fields which have to be addressed or filled for completing the report. Further, a template data structure may comprise one or more pull-down menu with items a user can select. As such, a template data structure may also be conceived as an input form or mask structuring the information to be provided for a given finding type.

The database may be a storage device such a cloud or local storage serving as an archive for template data structures. The database may provide an association linking template data structures to corresponding finding types. Thus, the lookup may comprise querying the database based on the predicted finding type. This may involve identifying the (appropriate) template data structure based on the predicted finding type and the association.

By automatically predicting the finding type based on at least the user input, a piece of information is gathered that can be fed into a structured reporting pipeline for the directed retrieval of template data structures. With that, a user is automatically provided with appropriate template data structures. In turn, the user is relieved from the burden of having to search for correct template data structure on her or his own in potentially vast databases. By contrast, the user is automatically provided with an actionable result he can immediately use in the downstream reporting workflow. Thereby, the template data structure is retrieved as a result of the specific inputs made by the user and, thus, reflects the immediate diagnostic context of the case and the user inputs. Accordingly, reviewing medical images and compiling structured medical reports on that basis can be rendered considerably more efficient. With that, one of the main drawbacks structured medical reports have in their practical implementation is addressed. Accordingly, as another advantage, also the acceptance of structured medical reporting in the field can be increased.

According to an aspect, the method further comprises:
- generating a medical report based on the identified template data structure and the user input, and
- providing the medical report.

According to an aspect, the step of providing comprises providing the template data structure to a user for editing the template data structure by the user, and the method further comprises:
- receiving a user input directed to editing the template data structure, and
- editing the template data structure based on the user input.

Accordingly, a user can edit the template data structure before it is transferred to the medical report. For instance, the user may fill data fields provided by the template data structure with further observations and measurement values. To this end, according to some examples, the user may select items from pull-down menus as provided for by the template data structure.

The method thus allows for a continued human machine interaction within which a user is continuously provided with information based on user actions. In addition, she or he can easily influence and modify the results generated.

According to an aspect, the user input is directed to generate a measurement of an image feature depicted in the representation, and the step of providing comprises pre-filling the template data structure with the measurement.

By automatically filling the template data structure, the user input is automatically transferred to the template data structure and, therewith, can be automatically transferred to the report. Hence, the user is provided with additional support.

According to an aspect, at least the steps of predicting the finding type and performing the lookup operation are performed at a server, the database is hosted by the server, and the step of providing comprises transmitting the template data structure to a client in data communication with the server and comprising the user interface.

In particular, the client may be a reading and reporting workstation, e.g., in the form of a laptop or desktop PC. The client may constitute the front end facing the user. The server may be conceived as the back end hosting the more computation heavy functionalities and providing access to databases for template data structures or medical image data sets. The server may be a local or cloud server. Data communication can be provided for by a medical or healthcare information systems which may be configured to communicate according to DICOM and/or HL7 standard. Health Level Seven or HL7 refers to a set of international standards for transfer of clinical and administrative data between software applications used by various healthcare providers.

By relying on a client-server setup, data, processing functions, and computation power may be shared between different clients rendering the setup more efficient and more readily scalable.

According to an aspect, the step of predicting the finding type is further based on the representation and/or the medical image data set and, in particular, based on image data comprised in the representation and/or the medical image data set.

According to some examples, the step of predicting the finding type comprises extracting, from the representation and/or the medical image data set, image data related to the user input and basing the prediction of the finding type on the related image data.

Specifically, an image patch of predetermined size may be extracted from the representation and/or the medical image data set which image patch is localized relative or around the location of the user input in the representation.

Accordingly, the image context of the user input may additionally be considered for predicting the finding type. With that, a more targeted prediction of the finding type may be achieved. To this end, the step of predicting may comprise an image processing step applied to image data comprised in the representation and/or the medical image data set.

According to an aspect, the method further comprises providing a prediction function configured to predict a finding type of a medical finding based on at least user inputs made with respect to representations of medical image data sets, wherein the step of predicting comprises applying the prediction function to the user input, and optionally, the prediction function comprises a machine-learned function trained to predict a finding type based on at least user inputs made with respect to representations of medical image data sets.

In particular, the prediction function and/or the machine-learned function may be configured to predict a finding type additionally based on image data of the representation and/or the medical image data set.

In general, a machine-learned function mimics cognitive functions that humans associate with other human minds. In particular, by training based on training data, the machine-learned function is able to adapt to new circumstances and to detect and extrapolate patterns. Other terms for machine-learned function, may be trained function, trained machine learning model, trained mapping specification, mapping specification with trained parameters, function with trained parameters, algorithm based on artificial intelligence, or machine learned algorithm.

In general, parameters of a machine-learned function can be adapted by means of training. In particular, supervised training, semi-supervised training, unsupervised training, reinforcement learning and/or active learning can be used. Furthermore, representation learning (an alternative term is "feature learning") can be used. In particular, the parameters of the machine-learned function can be adapted iteratively by several steps of training.

In particular, a machine-learned function can comprise a neural network, a support vector machine, a decision tree and/or a Bayesian network, and/or the trained function can be based on k-means clustering, Q-learning, genetic algorithms and/or association rules. In particular, a neural network can be a deep neural network, a convolutional neural network or a convolutional deep neural network. Furthermore, a neural network can be an adversarial network, a deep adversarial network and/or a generative adversarial network.

For instance, the machine-learned function may be configured to extract one or more features from the input data (that is: the user input and/or image data from the representation or the medical image data set and/or other/supplementary data) and map/classify these features into a feature space associated with different finding types for determining which finding type the extracted features indicate. Thus, the machine-learned function may comprise a feature extractor and a classifier. In particular, the feature extractor and the classifier may be implemented as a neural network, in particular, a convolutional neural network, with some network layers trained to extract features and other network layers being trained to provide a classification according to the most likely finding type(s).

The usage of machine-learned functions in general has the advantage that a more comprehensive and faster screening of the available information can be made. In this regard, machine-learned functions may identify patterns and attributes in the available data that are not accessible for a human.

According to an aspect, the machine-learned function has been trained based on medical image data sets of a patients comprising at least one medical finding with a verified finding type.

According to some examples, the medical image data sets respectively correspond to a key image of a medical image study.

By basing the training on medical image data sets or key images with known finding types, an efficient training can be performed. On the one hand, this data is readily available in healthcare information systems. On the other hand, this kind of data closely reflects the situation the machine-learned function will be confronted with when deployed.

According to an aspect, a computer-implemented method for providing a machine-learned function for predicting a finding type of a medical finding indicated in image data of a medical image data set is provided. The method comprises a plurality of steps. A first step is directed to provide a machine-learned function for predicting a finding type. A further step is directed to provide input training data comprising a medical image data set with one or more medical findings. A further step is directed to provide training output data comprising verified finding types of the medical findings of the medical image data set. A further optional step is directed to generate one or more hypothetical user inputs directed to the findings of the medical image data set. A further step is directed to input the medical image data set and/or the hypothetical user inputs into the machine-learned function to obtain one or more predicted finding types of the medical findings comprised in the medical image data set. A further step is directed to compare the predicted finding types with the output training data. A further step is directed to adjust the machine-learned function based on the step of comparing. A further step is directed to provide the adjusted machine-learned function.

Hypothetical user inputs may, for instance, be generated by simulating user inputs that will likely be generated by users in the field, such as applying a measurement tool, highlighting or marking a certain pattern, or any other likely interaction with the available data upon reviewing a patient case.

With the proposed training method, a realistic scenario may be generated for the machine-learned function and, hence, a good model performance may be ensured.

According to another aspect, a training system for providing a machine-learned function for predicting a finding type of a medical finding indicated in image data of a medical image data set is provided. The system comprises an interface embodied for receiving the machine learned function and the input and output training data. The system further comprises a computing unit configured to run the machine-learned function. The computing unit is further configured to carry out the training steps according to the method for providing a machine-learned function and its aspects as previously described.

According to an aspect, the method further comprises obtaining supplementary medical information of the patient, wherein the step of predicting is further based on the supplementary medical information.

According to some examples, the supplementary information may be non-image data of the patient. Obtaining the supplementary information may comprise querying a healthcare information system such as a HIS (hospital information system), a LIS (laboratory information system), EMR-system (electronic medical record system) for supplementary information of the patient. Such supplementary information may be obtained in the form of one or more EMR-files, for instance. Further, querying healthcare information systems may be based on a patient identifier such as an ID or the patient name, electronically identifying the patient in the system.

According to some examples, the prediction function / the machine-learned function is further configured to predict a finding type of medical findings additionally based on the supplementary medical information.

By basing the prediction on supplementary information, a more precise prediction can be achieved.

According to some examples, the supplementary information comprises one or more of the following elements:
- a prior medical report of the patient, and/or
- a-priori knowledge of at least one type of medical problem the patient is suspected to have, and/or
- an indication of a diagnostic task to be performed based on the medical image data set for the patient, and/or
- a medical guideline applicable for the patient, and/or
- an electronic health record of the patient.

According to some examples, the electronic health record may comprise the patient history of the patient including any pre-existing illnesses, comorbidities, risk factors, referral letters, demographic information such as age or gender, and the like.

With the supplementary information mentioned above, the diagnostic context can be pinpointed which enables a more precise prediction of the finding type.

According to some examples, the prior medical report comprises at least one indication of a finding type previously reported for the patient, the method further comprises extracting the indication of the finding type from the medical report, and the step of predicting is further based on the extracted indication of the finding type.

Extracting the indication may comprise identifying one or more relevant data structures in the prior medical report (if the prior medical report is a structured medical report) or applying a natural language processing function, e.g., configured to identify one or more key words in free text. The supplementary information may also be processed in the same way to extract indications of previously reported finding types.

By making use of finding types previously reported, the possible finding types can be narrowed down and the prediction can be improved.

According to some examples, the prediction function / the machine-learned function is further configured to extract indications of finding types from medical reports (the supplementary information), and the step of extracting comprises applying the prediction function / the machine-learned function to the prior medical report.

According to an aspect, the method comprises identifying, from a plurality of reference medical images of reference patients different than the patient, at least one similar medical image based on degrees of similarity between the representation and individual reference medical images. Thereby each reference medical image comprises one or more findings with a verified finding type, and the step of predicting is further based on at least one verified finding type of the identified at least one similar medical image.

The reference medical images may be stored or held available in a corresponding database. The degree of similarity may, in particular, be based on an image similarity measuring/quantifying how similar the representation and the respective reference medical image are. In particular, the reference medical images may be key images that have been generated from medical image data sets of previous patients. In particular, such key images may have been selected by a user as significant for the diagnostic workflow.

In other words, finding types are being predicted based on cases which are similar to the case at hand. With that, the method can make use of already existing knowledge and the accuracy of the predicted finding types can be improved.

According to some examples, the prediction function / the machine-learned function is further configured to identify similar medical images, and the step of identifying comprises applying the prediction function / the machine-learned function to the representation and the reference medical images.

According to some examples, the step of identifying the at least one similar medical image comprises:
- extracting an image descriptor from the representation;
- respectively extracting a corresponding image descriptor from the plurality of reference medical images, and
- determining, for each one of the plurality of reference medical images, a similarity metric indicative of the degree of similarity between the image descriptor of the representation and the respective image descriptor of the respective reference medical image.

In some examples, an image descriptor may be a vector representing or characterizing the underlying image data of either the representation or the reference medical images. A feature vector as herein described may be based on image data and non-image data. In some examples, the image descriptor may be determined by encoding the representation or the reference medical images using a particular image descriptor encoding algorithm (also referred to as "vectorizer)".

Features of a feature vector may be visual features, such as a visual manifestation of a medical abnormality, a pattern, an anatomy, a medical landmark and so forth as indicated by the image of the respective medical image series. Further, a feature vector may comprise non-image features, for instance relating to the imaging modality or imaging parameters used. The image descriptors may be calculated on-line, e.g., upon the prediction of a finding type. Alternatively, image descriptors of the reference medical images may be held available as pre-generated data items stored in a database, e.g., alongside the reference medical images.

According to some examples, the degrees of similarity may be calculated by applying a similarity metric representing how similar the representation is to a reference medical image. Another expression for similarity metric may be distance metric. In some examples, the similarity metric may be configured to quantify a distance in vector or feature space between the image descriptor of the representation and the image descriptor of a respective one of the reference medical images. According to some examples, predetermined mathematical functions may be used to calculate such a distance such as the cosine similarity or the Euclidean distance and so forth. According to other examples, the similarity or distance metric may comprise a learned metric which has been derived by machine learning. According to some implementations, the extraction of the image descriptors and/or the evaluation of a similarity metric as whole may be performed by the machine-learned function.

The selection of similar images based on feature vectors allows for a flexible selection. For example, the comparison of feature vectors in the feature space may be more robust and/or flexible with respect to non-exact matches between parameters, e.g., as compared to attempting to match images directly. Accordingly, the prediction of the finding types can be improved. In turn, an applicability of the template data structures provided can ensured and the support for the user in the diagnostic workflow can be improved.

According to an aspect, the method further comprises determining an anatomical location the user input is directed to based on the user input and the representation and/or the medical image data set. Further, the step of predicting the finding type is further based on the anatomical location.

According to an aspect, the anatomical location comprises one or more of the following elements:
- an indication of an organ or organ part the user input is directed to,
- a slice within the medical image data set, the representation corresponds to, and/or
- a relation of the user input to one or more anatomical landmarks.

With the anatomical location, information may be retrieved indicating the anatomic context of the user input and, therewith, the medical finding. With that, the group of potentially applicable finding types can be narrowed down yielding better results in the prediction stage as well as in the downstream reporting process.

According to an aspect, the method further comprises filtering the supplementary medical information based on the anatomical location, wherein the step of predicting is further based on the filtered supplementary medical information.

In particular, the step of filtering may comprise identifying, within the supplementary medical information, information relating to the anatomical location.

In other words, those pieces of information in the (potentially vast) supplementary medical information are identified which bear a certain relevance for the anatomical location currently considered by the user. This may lead to a simplified processing and better results.

According to an aspect, the method further comprises obtaining one or more displaying settings applied by the user for the representation upon displaying the representation, and the step of predicting the finding type is further based on the displaying settings.

According to an aspect, the displaying settings comprise one or more of the following settings:
- an organ segmentation applied to the representation,
- an intensity windowing applied to the representation,
- contrast and/or brightness adjustments applied to the representation
- a look-up table applied to the representation,
- an auto-view setting applied to the representation,
- a viewing plane applied to the representation, and/or
- a zoom level or panning applied to the representation.

Taking CT image data as an example, the representation may be intensity filtered. Each voxel of a CT image data set usually has an intensity value that represents the attenuation of X-rays at the corresponding position in the volume as determined from the CT measurements (commonly measured in Hounsfield or HU units). Due to this relation, a kind of segmentation can be performed based solely on a thresholding of the voxel intensity values. For example, all voxels having intensity values in a particular range may be considered to represent bone. The corresponding range is called the "bone window". Other windows may relate to carve out soft tissue or lung parts. In the art, these windows are commonly referred to as HU-windows.

An auto-viewing setting may, in particular, relate to a setting for a particular diagnostic task or a particular organ. The auto-viewing setting may be provided by the user interface for selection by the user. Since the auto-viewing settings are specific to a certain diagnostic task or anatomy, their knowledge may provide insights into the likely finding types.

In particular, the viewing plane may be selected from an axial or sagittal or coronal viewing plane. Generally speaking, it relates to the viewing plane under which the medical image data set (or the underlying image volume) is being looked at. In the diagnostic workflow, the viewing direction already is an indication for the type of findings a user is interested in.

In general, taking display or viewing settings into account upon predicting finding types is beneficial, as such display settings provide valuable insights into the kind of organs, anatomies and medical findings, a user is interested in.

Thus, the prediction of the finding types and, therewith, the ensuing reporting workflow may be improved.

According to some implementations, the step of determining an anatomical location may further be based on the display settings.

According to an aspect, the method further comprises obtaining at least one imaging parameter of the medical image data set, the imaging parameter relating to settings used during acquisition and/or pre- or post-processing of the medical image data set wherein the step of predicting is further based on the imaging parameter.

According to an aspect, the imaging parameter comprises one or more of the following parameters:
- a patient position during the image acquisition process,
- an image acquisition protocol used for acquiring raw data based on which the medical image data is generated, and/or
- an image reconstruction protocol used for generating the medical image data set based on raw data.

The acquisition protocol may relate to the kind of medical imaging modality used for acquiring the medical image data or the underlying raw data. For instance, the acquisition protocol may specify if an MRI system or a CT system has been used. Further, the acquisition protocol may relate to settings used for the medical imaging modality during the acquisition. Here, different physical parameters adjusted at the imaging modality as well as parameters relating to the controlled adjustment of different physiological conditions of the patient may be comprised. Taking an MRI system as an example, this may further amount to the MR pulse sequence used.

The reconstruction protocol may relate to the reconstruction algorithm and the corresponding settings used for processing the acquired raw data so as to provide the medical image data set. Taking the CT imaging process as an example, the reconstruction protocol may specify the kernel (or convolution algorithm) used. The kernel refers to the process used to modify the frequency contents of projection data prior to back projection during image reconstruction in a CT scanner. This process corrects the image by reducing blurring. The kernel affects the appearance of image structures by sharpening the image. Different kernels have been developed for specific anatomical applications including soft tissue (standard kernel) and bone (bone kernel).

Taking imaging parameters into account upon predicting finding types is beneficial since the imaging parameters provide additional insights into the kind of organs, anatomies, and medical findings, a medical image data set can indicate. Thus, the prediction of the finding types and therewith the ensuing reporting workflow may be improved by considering this information in the step of predicting the finding type.

According to an aspect, the user input is directed to generate a measurement of an image feature depicted in the representation, and the step of predicting the finding type is further based on the measurement.

For instance, the measurement may be a volume, surface, angle, or distance in the representation. Further, the measurement may involve defining a region of interest in the representation. In addition, the measurement may be based on image data related to the user input. For instance, an intensity profile (e.g., measured in Hounsfield units) of such image data may indicate if a certain medical finding relates to a cyst or a malignancy.

As the measurement may indicate the type of finding (for instance, a measured distance may indicate a different type of finding as compared to a volume measurement), taking the measurement into account upon predicting the finding type can improve the outcome.

According to an aspect, generating the measurement involves selecting an image measurement tool from a plurality of available image measurement tools by the user, and the step of predicting the finding type is further based on the measurement tool selected by the user.

In general, there will be different kinds of measurement tools available for the user. For instance, such tools may be geometry measurement tools, volume measurement tools, image processing tools, and/or computer aided detection tools. Accordingly, the kind of tool invoked by the user also indicates the finding type of a corresponding medical finding identified with the tool. Thus, using this information in the prediction step may further improve the outcome.

According to an aspect, predicting a finding type of the medical finding comprises selecting the finding type from a predetermined set of a plurality of candidate finding types.

Specifically, the method may further comprise obtaining a plurality of different sets of a plurality of candidate finding types, and selecting the predetermined set from the plurality of sets based on one or more of: the medical image data set, the supplementary medical information, the anatomical location, the display settings, the imaging parameters, and/or the at least one similar image.

In other words, a plurality of preconfigured sets of candidate finding types is provided which are selected according to the circumstances of the case. As such, the sets may be conceived as buckets into which findings types may be sorted or grouped. For instance, a set of candidate finding types for the lung may be provided. If the information gathered indicates that the user is going to review the lung of the patient, this set may be specifically selected. In turn, this pre-selection may facilitate the prediction process as the choice of potential medical findings has already been narrowed down.

According to an aspect, each of the plurality of sets is associated to a diagnostic use case, and the step of selecting the predetermined set comprises:
- determining an actual diagnostic use case for the medical image date set based on one or more of: the medical image data set, the supplementary medical information, the anatomical location, the display settings, the imaging parameters, and/or the at least one similar image, and
- selecting the predetermined set by comparing the actual diagnostic use case with the associated diagnostic use case.

By associating the finding types to a diagnostic use cases, an efficient pre-selection of the finding types can be effected. Thereby, the actual diagnostic use case is determined by identifying the most likely applicable diagnostic use case from the associated use cases.

According to an aspect, the step of predicting comprises:
- predicting a plurality of likely finding types for selection by the user,
- providing the plurality of likely finding types to the user,
- receiving a user input directed to select one of the likely finding types,
- providing the finding type based on the user selection.

Accordingly, the final selection of the appropriate finding type may be performed by the user. Accordingly, the user is provided with additional means to influence and control the processing in terms of a continued human-machine interaction for picking the right template data structure in a reporting workflow. Still, with the automated initial selection of the likely finding types, a user is provided with a considerable amount of assistance.

According to some examples, the likely finding types may relate to differential diagnosis for the patient. With that, differential diagnosis may be systematically brought to the attention of the user for further consideration.

According to an aspect, the method further comprises adapting the prediction function / the machine-learned function based on the user input directed to select.

According to an aspect, the step of selecting comprises:
- providing the predicted finding type to the user,
- receiving a user input directed to confirm or reject the predicted finding type, and
- the method further comprises adapting the prediction function / the machine-learned function based on the user input directed to confirm or reject.

By adapting the prediction function / the machine-learned function based on feedback from the user, the algorithms involved in the prediction can be continuously improved. This improves the accuracy and reliability of the method.

According to an aspect, a system for providing a template data structure for a medical report is provided. The system comprises an interface unit, a computing unit, and a database. The database is configured to store a plurality of report template data structures for medical reports, wherein each report data template structure corresponds to a distinct finding type of a medical finding. The interface unit is configured to receive a medical image data set depicting a body part of a patient, to forward a representation of the medical image data set for displaying to a user, to receive a user input from the user directed to indicate a medical finding in the representation, and to provide the template data structure to the user. The computing unit is configured to generate the representation of the medical image data set for displaying to the user, to predict a finding type of the medical finding based on the user input, and to perform a lookup operation in the report database to identify a template data structure corresponding to the predicted finding type.

The computing unit may be realized as a data processing system or as a part of a data processing system. Such a data processing system can, for example, comprise a cloud-computing system, a computer network, a computer, a tablet computer, a smartphone and/or the like. The computing unit can comprise hardware and/or software. The hardware can comprise, for example, one or more processors, one or more memories and combinations thereof. The one or more memories may store instructions for carrying out the method steps according to the invention. The hardware can be configurable by the software and/or be operable by the software. Generally, all units, sub-units, or modules may at least temporarily be in data exchange with each other, e.g., via a network connection or respective interfaces. Consequently, individual units may be located apart from each other.

The interface unit may comprise an interface for data exchange with a local server or a central web server via internet connection for receiving the medial image data sets. The interface unit may be further adapted to interface with one or more users of the system, e.g., by displaying the result of the processing by the computing unit to the user (e.g., in a graphical user interface) or by allowing the user to adjust parameters for image processing or visualization.

According to other aspects, the invention further relates to an image analysis system comprising the above system and a medical image system (or medical/healthcare information system) configured to acquire, store and/or forward medical images. Thereby, the interface unit is configured to receive the medical image data sets form the medical image system.

According to some examples, the medical image system comprises one or more archive stations for storing medical images which may be realized as a cloud storage or as a local or spread storage, e.g., as a PACS (Picture Archiving and Communication System). Further, the medical image system may comprise one or more medical imaging modalities, such as a computed tomography system, a magnetic resonance system, an angiography (or C-arm X-ray) system, a positron-emission tomography system, a mammography system, or the like.

According to other aspects, the systems are adapted to implement the inventive method in their various aspects for providing a candidate medical finding. The advantages described in connection with the method aspects may also be realized by the correspondingly configured systems' components.

According to another aspect, the present invention is directed to a computer program product comprising program elements which induce a computing unit of a system configured to provide a template data structure for a medical report to perform the steps according to one or more of the above method aspects, when the program elements are loaded into a memory of the computing unit.

According to another aspect, the present invention is directed to a computer-readable medium on which program elements are stored that are readable and executable by a computing unit of a system for providing a template data structure for a medical report according to one or more method aspects, when the program elements are executed by the computing unit.

The realization of the invention by a computer program product and/or a computer-readable medium has the advantage that already existing providing systems can be easily adapted by software updates in order to work as proposed by the invention.

The computer program product can be, for example, a computer program or comprise another element next to the computer program as such. This other element can be hardware, e.g., a memory device, on which the computer program is stored, a hardware key for using the computer program and the like, and/or software, e.g., a documentation or a software key for using the computer program. The computer program product may further comprise development material, a runtime system and/or databases or libraries. The computer program product may be distributed among several computer instances.

Characteristics, features and advantages of the above-described invention, as well as the manner they are achieved, become clearer and more understandable in the light of the following description of embodiments, which will be described in detail with respect to the figures. This following description does not limit the invention on the contained embodiments. Same components, parts or steps can be labeled with the same reference signs in different figures. In general, the figures are not drawn to scale. In the following:
- FIG. 1: schematically depicts a system for providing a template data structure according to an embodiment,
- FIG. 2: schematically depicts a method for providing a template data structure according to an embodiment,
- FIG. 3: schematically depicts a data flow diagram in a method for providing a template data structure according to an embodiment,
- FIG. 4: schematically depicts optional method steps in a method for providing a template data structure according to an embodiment,
- FIG. 5: schematically depicts optional method steps in a method for providing a template data structure according to an embodiment,
- FIG. 6: schematically depicts optional method steps in a method for providing a template data structure according to an embodiment,
- FIG. 7: schematically depicts a prediction function for predicting a finding type according to an embodiment,
- FIG. 8: schematically depicts a method for providing a prediction function for predicting a finding type according to an embodiment, and
- FIG. 9: schematically depicts a system for providing a prediction function according to an embodiment.

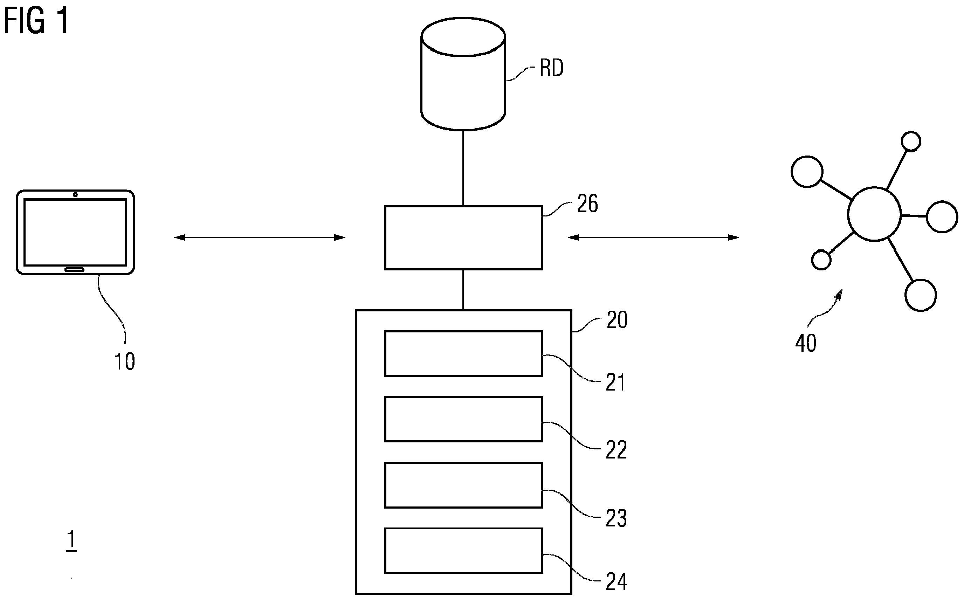

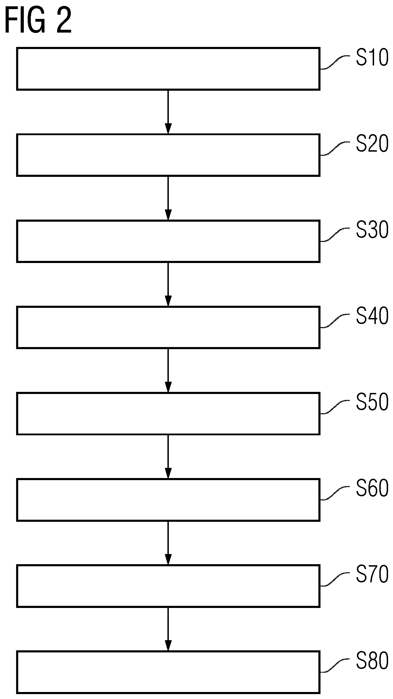

Figure 1 depicts a system 1 for providing a template data structure TDS for a medical report MER. In this regard, system 1 is adapted to perform the methods according to one or more embodiments, e.g., as further described with reference to Figures 2 to 6. A user of system 1, according to some examples, may generally relate to a healthcare professional such as a physician, clinician, technician, radiologist, pathologist and so forth.

System 1 comprises a user interface 10 (as part of the interface unit) and a processing system 20 (as part of the computing unit). Further, system 1 may comprise or be connected to a medical information system 40. The medical information system 40 may generally be configured for acquiring and/or storing and/or forwarding medical image data sets MIDS and supplementary (non-image) information. For instance, medical information system 40 may comprise one or more archive/review station (not shown) for medical image data sets MIDS. The archive/review stations may be embodied by one or more databases. In particular, the archive/review stations may be realized in the form of one or more cloud storage modules. Alternatively, the archive/review stations may be realized as a local or spread storage, e.g., as a PACS (Picture Archiving and Communication System). According to some examples, medical information system 40 may also comprise one or more medical imaging modalities (not shown), such as a computed tomography system, a magnetic resonance system, an angiography (or C-arm X-ray) system, a positron-emission tomography system, a mammography system, an X-ray system, or the like.

Medical image data sets MIDS may be three-dimensional image data sets acquired, for instance, using an X-ray system, a computed tomography system or a magnetic resonance imaging system or other systems. The image information may be encoded in a three-dimensional array of m times n times p voxels. Medical images IM may include a plurality of image slices which are stacked in a stacking direction to span the image volume covered by the medical image data sets MIDS.

Further, medical image data sets MIDS may comprise two-dimensional medical image data with the image information being encoded in an array of m times n pixels. According to some examples, these two-dimensional medical image data sets MIDS may have been extracted from three-dimensional medical image data sets. According to other examples, two-dimensional medical image data sets MIDS may have been generated by dedicated imaging modalities such as slide scanners used in digital pathology.

An ensemble of voxels or pixels may be designated as image data of the respective medical image data sets MIDS in the following. In general, any kind of imaging modalities and scanners may be used for acquiring such image data. Generally, radiology medical image data sets MIDS show a body part or an anatomical region or an anatomic object of a patient which may comprise various anatomies and organs. Considering the chest area as a body part, medical image data sets MIDS might, for instance, depict the lung lobes, the rib cage, the heart, lymph nodes, and so forth. By contrast, pathology medical imaged data sets show a tissue section of the patient.

Medical image data sets MIDS may be formatted according to the DICOM format. DICOM (=Digital Imaging and Communications in Medicine) is an open standard for the communication and management of medical imaging information and related data in healthcare informatics. DICOM may be used for storing and transmitting medical images and associated information enabling the integration of medical imaging devices such as scanners, servers, workstations, printers, network hardware, and picture archiving and communication systems (PACS). It is widely adopted by clinical syndicates, hospitals, as well as for smaller applications like doctors' offices or practices. A DICOM data object consists of a number of attributes, including items such as the patient's name, ID, etc., and also special attributes containing the image pixel data and metadata extracted from the image data.

Supplementary information may be any data providing additional information relating to the patient and/or the medical image data set MIDS. The supplementary information may comprise image data such as other medical image data sets MIDS of the patient which were, for instance, at an earlier point in time than the medical image data set MIDS under consideration. Further the supplementary information may comprise non-image data or data with mixed-type contents comprising medical images and non-image contents such as text. Non-image data may relate to non-image examination results such as lab data, vital signs records (comprising, e.g., ECG data, blood pressure values, ventilation parameters, oxygen saturation levels) and so forth. Moreover, the supplementary information may comprise structured and unstructured reports relating to prior examinations of the patient. Further, non-image data may comprise personal information of the patient such as gender, age, weight, insurance details, and so forth.

The supplementary information may be available in the form of one or more electronic medical reports of the patient. The supplementary information may be stored in the healthcare information system 40. For instance, the supplementary information may be stored in dedicated databases of the healthcare information system 40 such as laboratory information system (LIS) or an electronic health/medical record database.

User interface 10 may comprise a display unit and an input unit. User interface 10 may be embodied by a mobile device such as a smartphone or tablet computer. Further, user interface 10 may be embodied as a workstation in the form of a desktop PC or laptop. The input unit may be integrated in the display unit, e.g., in the form of a touch screen. As an alternative or in addition to that, the input unit may comprise a keyboard, a mouse or a digital pen and any combination thereof. The display unit may be configured for displaying a representation R of the medical image data set MIDS, receiving any user input INP, e.g., for identifying a medical finding MF, and displaying any results and images derived therefrom in the course of the method execution such as the predicted finding type FT or the template data structure TDS.

User interface 10 may further comprise an interface computing unit configured to execute at least one software component for serving the display unit and the input unit in order to provide a graphical user interface for allowing the user to select a target patient's case to be reviewed and making various inputs INP. In addition, the interface computing unit may be configured to communicate with medical information system 40 or processing system 20 for receiving the medical image data sets MIDS and any supplementary information. The user may activate the software component via user interface 10 and may acquire the software component, e.g., by downloading it from an internet application store. According to an example, the software component may also be a client-server computer program in the form of a web application running in a web browser. The interface computing unit may be a general processor, central processing unit, control processor, graphics processing unit, digital signal processor, three-dimensional rendering processor, image processor, application specific integrated circuit, field programmable gate array, digital circuit, analog circuit, combinations thereof, or other now known devices for processing image data. User interface 10 may also be embodied as a client.

Processing system 20 may comprise sub-units 21-24 configured to process the medical image data sets MIDS in order to provide one or more template data structures TDS based on medical findings MF indicated by the image data comprises in the medical image data sets MIDS, and, optionally, to provide a platform for editing the template data structure TDS and drawing up a medical report MER on that basis.

Processing system 20 may be a processor. The processor may be a general processor, central processing unit, control processor, graphics processing unit, digital signal processor, three-dimensional rendering processor, image processor, application specific integrated circuit, field programmable gate array, digital circuit, analog circuit, combinations thereof, or other now known device for processing image data. The processor may be single device or multiple devices operating in serial, parallel, or separately. The processor may be a main processor of a computer, such as a laptop or desktop computer, or may be a processor for handling some tasks in a larger system, such as in the medical information system or the server. The processor is configured by instructions, design, hardware, and/or software to perform the steps discussed herein. The processing system 20 may be comprised in the user interface 10. Alternatively, processing system 20 may comprise a real or virtual group of computers like a so called 'cluster' or 'cloud'. Such server system may be a central server, e.g., a cloud server, or a local server, e.g., located on a hospital or radiology site. Further, processing system 20 may comprise a memory such as a RAM for temporally loading the medical image data sets MIDS. According to some examples, such memory may as well be comprised in user interface 10.

Sub-unit 21 is a data retrieval module or unit. It is configured to access and search the medical information system 40 for the medical image data sets MIDS. Specifically, sub-unit 21 may be configured to formulate search queries and parse them to medical information system 40. In the same manner, sub-unit 21 may be configured to retrieve supplementary information for the patient.

Sub-unit 22 may be configured as a user interaction module or unit. Sub-unit 22 may be configured to provide a representation R for displaying to the user via the user interface 10. The representation R can be in the form of a rendering. Further, sub-unit 22 may be configured to identify a medical finding MF in the representation R (and, therewith, in the medical image data set MIDS) based on a corresponding input INP by the user. For instance, sub-unit 22 may be configured to provide a corresponding tool, the user may activate via the user interface 10. After the tool has been activated, user inputs INP such as speech, gesture, eye movement, handling of input devices such as computer mouses, etc. may be evaluated to derive the location of a medical finding MF. According to an example, such a user input INP may designate a point or group of points in the representation R which is then further processed to define the medical finding MF. According to other examples, the tool may be a measurement tool with which the user may obtain a measurement from the representation R. The measurement may relate to a volume, surface, angle, distance, etc.

Further, sub-unit 22 may be configured to also receive and process other kinds of user inputs to control the method and allow for a continued and guided human-machine interaction. Such user inputs may be directed to select an appropriate finding type FT from a plurality of finding types provided by the system, to input displaying settings for adjusting the representation R, or to edit the template data structure TDS.

Sub-unit 23 can be conceived as a prediction module or unit. It is configured to process the medical image data set MIDS and/or the user input INP in order to predict one or more likely finding types FT of a medical finding MF indicated by the user input INP. Specifically, sub-unit 23 may be configured to use the available information to come up with an educated guess for the finding type FT. To predict finding types FT, sub-unit 23 may be configured to run an accordingly configured prediction function PF according to some examples.

Sub-unit 24 may be conceived as a reporting module or unit. Specifically, sub-unit 24 may be configured to query the report database RD for appropriate template data structures TDS corresponding to the finding type FT. For instance, this may be done based on an electronic association linking finding types FT to corresponding template data structures TDS. Further, sub-unit 24 may be configured to pre-fill the template data structure TDS based on user inputs INP. For instance, sub-unit 24 may be configured to include measurements in the template data structure TDS. Moreover, sub-unit 24 may be configured to transfer the template data structure TDS into a medica report MER format which can be forwarded and/or stored for later use. To this end, sub-unit 24 may be configured to combine a plurality of template data structures TDS into one medical report MER.

The designation of the distinct sub-units 21-24 is to be construed by way of example and not as a limitation. Accordingly, sub-units 21-24 may be integrated to form one single unit (e.g., in the form of "the computing unit") or can be embodied by computer code segments configured to execute the corresponding method steps running on a processor or the like of processing system 20. The same holds true with respect to the interface computing unit. Each sub-unit 21-24 and the interface computing unit may be individually connected to other sub-units and/or other components of the system 1 where data exchange is needed to perform the method steps. For example, sub-unit 21 may be connected via an interface 26 to medical information system 40 for retrieving the medical image IM. Likewise, interface 26 may connect the sub-units 21 to 24 to the user interface 10 for forwarding the results of the computation to the user and collect user inputs.

Processing system 20 and the interface computing unit together may constitute the computing unit of the system 1. Of note, the layout of this computing unit, i.e., the physical distribution of the interface computing unit and sub-units 21-24 is, in principle, arbitrary. For instance, sub-unit 22 (or individual elements of it or specific algorithm sequences) may likewise be localized in user interface 10. The same holds true for the other sub-units 21-24. Specifically, processing system 20 may also be integrated in user interface 10. As already mentioned, processing system 20 may alternatively be embodied as a server system, e.g., a cloud server, or a local server, e.g., located on a hospital or radiology site. According to such implementation, user interface 10 could be designated as a "frontend" or "client" facing the user, while processing system 20 could then be conceived as a "backend" or server. Communication between user interface 10 and processing system 20 may be carried out using the https-protocol, for instance. The computational power of the system may be distributed between the server and the client (i.e., user interface 10). In a "thin client" system, the majority of the computational capabilities exists at the server. In a "thick client" system, more of the computational capabilities, and possibly data, exist on the client.

The report database RD is a storage device such a cloud or local storage serving as an archive for template data structures TDS and, optionally, medical reports MER.

Thereby, a template data structure TDS may be seen as a building block for a medical report MER. Template data structures TDS may be configured for editing by the user via user interface 10. Further, template data structures TDS may be configured to be included in a medical report MER. Template data structures TDS may comprise one or more data fields DF into which information specific for the patient and/or the underlying medical finding MF may be specified. The data fields DF may be empty fields or placeholders for various kinds of data such as text, measurement values or images. According to some examples, template data structures TDS comprise one or more pull down menus with entries a user can select from.

A template data structure TDS may be specific to a certain finding type FT. In other words, template data structures TDS for different finding types TF may be different. For instance, they may differ in the number and type of data fields DF.

Individual components of system 1 may be at least temporarily connected to each other for data transfer and/or exchange. User interface 10 communicates with processing system 20 via (data) interface 26 to exchange, e.g., medical image data sets MIDS, the template data structure TDS, the final medical report MER, or any user input INP made. For example, processing system 20 may be activated on a request-base, wherein the request is sent by user interface 10. Further, processing system 20 may communicate with medical information system 40 in order to retrieve a target patient's case. As an alternative or in addition to that, user interface 10 may communicate with medical information system 40 directly. Medical information system 40 may likewise be activated on a request-base, wherein the request is sent by processing system 20 and/or user interface 10. Data interface 26 for data exchange may be realized as hardware- or software-interface, e.g., a PCI-bus, USB or fire-wire. Data transfer may be realized using a network connection. The network may be realized as local area network (LAN), e.g., an intranet or a wide area network (WAN). Network connection is preferably wireless, e.g., as wireless LAN (WLAN or Wi-Fi). Further, the network may comprise a combination of different network examples. Interface 26 for data exchange together with the components for interfacing with the user be regarded as constituting an interface unit of system 1.

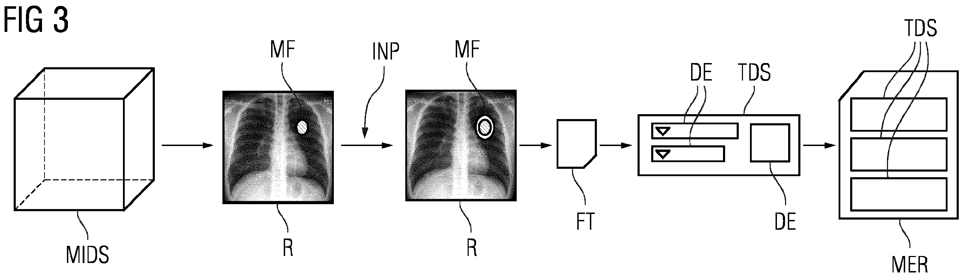

Figure 2 depicts a method for providing a template data structure TDS according to an embodiment. Corresponding data streams are illustrated in Figure 3. The method comprises several steps. The order of the steps does not necessarily correspond to the numbering of the steps but may also vary between different embodiments of the present invention. Further, individual steps or a sequence of steps may be repeated.

In a first step S10, the medical image data set MIDS is received. This may involve selecting the medical image data set MIDS from a plurality of cases, e.g., stored in the medical information system 40. The selection may be performed manually by a user, e.g., by selecting appropriate image data in a graphical user interface running in the user interface 10.

Alternatively, the medical image data set MIDS may be provided to the computing unit by a user by way of uploading the medical image data set MIDS to the computing unit.

At step S20, a representation R of the medical image data set MIDS for display to the user via the user interface 10 is rendered. As shown in Figure 3, the representation R may show a medical finding MF.

At step S30, the representation R is provided to the user via the user interface 10. The user may adjust the displaying settings for displaying the representation R. For instance, such displaying settings may comprise one or more segmentation masks applied by the user, one or more intensity windows or filters applied by the user, one or more contrast, brightness, intensity settings applied by the user, one or more zooming, panning or viewing plane settings applied by the user, and the like.

That followed, at step S40, a user input INP is received. The user input INP received in step S40 may be directed to the medical finding MF visible in the representation R. As can be seen in Figure 3, the user input INP may be directed to encircle the finding and or derive a corresponding measurement such as the volume or size. As mentioned, the user input INP may also involve using a measurement tool to identify/measure the medical finding MF. According to further examples, the user input may also comprise activating an automated detection function configured to identify medical findings MF in medical image data sets MIDS.

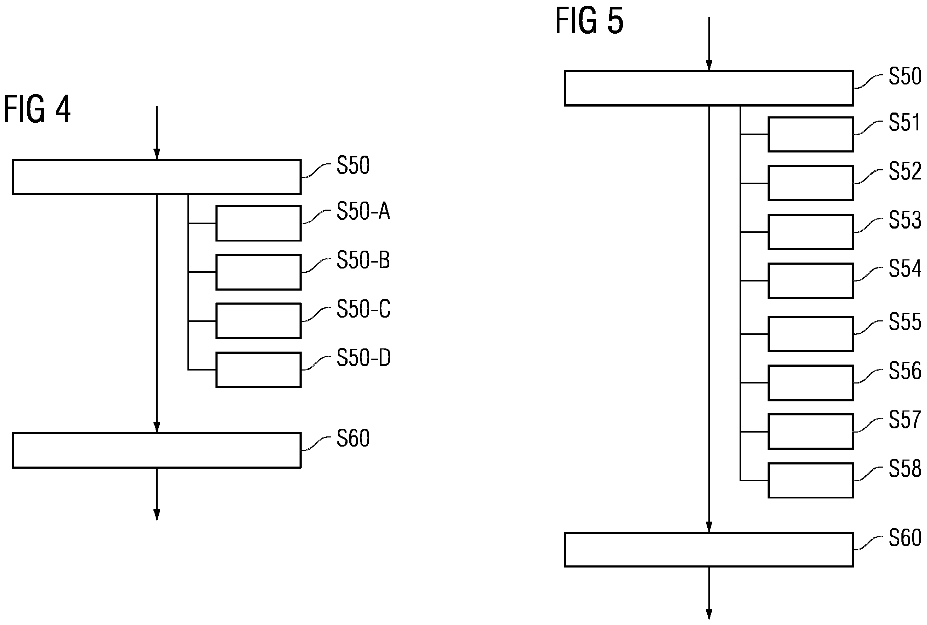

At step S50, the finding type FT of the medical finding MF identified by the user input INP is predicted. The prediction may be based on the user input INP and/or image data comprised in the representation R or the medical image data set MIDS. In addition, also further information may be taken into account such as the supplementary information or the displaying settings. Further details will be provided in connection with Figure 5.

In the step of predicting, a plurality of finding types FT may be predicted which come into question. This set may be seen as a group of likely finding types FT from which a user can pick the "final" finding type FT for the ensuing reporting workflow. Further details regarding this semi-automatic selection process will be given in Figure 4.

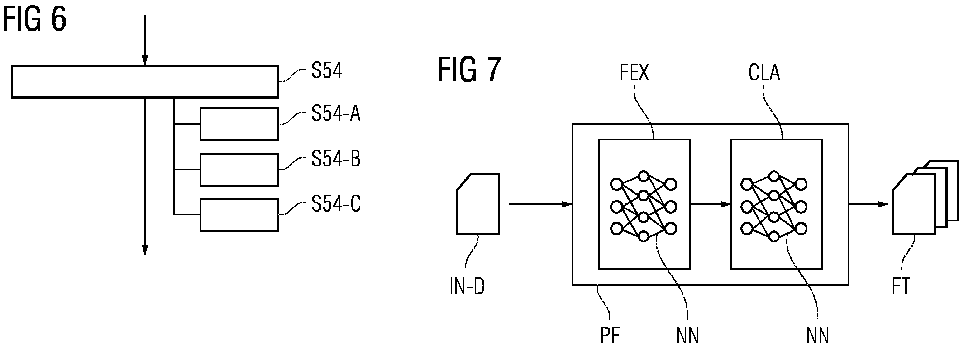

The prediction of the finding type FT may comprise inputting the available input data such as the user input INP, the representation, and any other relevant data in a prediction function PF which may comprise machine-learned modules (c.f. Figures 7 to 9).

According to an embodiment, step S70 may comprise a confirmation interaction with the user. Here, the user may evaluate whether or not the finding type FT is correct. To this end, the user interface 10 may be configured to receive a user input direct to decline or accept the finding type FT. If the finding type is declined, another finding type may be predicted.

At step S60, at least one template data structure TDS is retrieved from the report database RD which matches the predicated finding type FT. To this end, a lookup operation may be performed in the reporting database RP for template data structures TDS corresponding to the predicted finding type FT. Specifically, an association linking the template data structures TDS in the reporting database RD with applicable finding types FT may be used to find correct template data structures TDS.

At step S70, the template data structure TDS identified at step S60 is provided. Providing may mean that the template data structure TDS is displayed to the user via user interface 10. In particular, the template data structure TDS may be provided such that a user can edit the template data structure TDS, e.g., by filling data fields DF as provided for in the template data structure TDS.

Further, step S70 may comprise pre-filling distinct data fields DF of the template data structure TDS with pieces of information already available. In particular, this may comprise any measurements generated based on the user inputs INP.

At optional step S80, a medical report MER is generated based on the template data structure TDS. This may involve receiving user inputs directed to edit the template data structure TDS from the user via user interface 10. This may comprise entering or changing contents in the template data structure TDS. For the medical report MER, the template data structure TDS thus finalized by the user may be combined with other template data structures TDS related to other medical findings MF which may have been provided by similar workflows. The resulting medical report MER may then be forwarded to other systems or users, e.g., via the healthcare information system 40. Further, the resulting medical report MER may be archived, e.g., in the reporting database RD or in the healthcare information system 40.

Figure 4 depicts optional method steps for allowing a user to select a finding type FT from a set of automatically retrieved finding types TF which come into question after step S50. The order of the steps does not necessarily correspond to the numbering of the steps but may also vary between different embodiments of the present invention. Further, individual steps or a sequence of steps may be repeated.

At first, in step S50-A a set of candidate finding types FT is provided. The candidate finding types FT may be the most likely finding types FT based on the prediction as described in step S50. The most likely finding types FT may be those finding types FT having the greatest confidence score based on the information available.

At step S50-B the candidate finding types FT are provided to the user. For instance, this may involve displaying the finding types FT to the user in user interface 10. According to some examples, any confidence scores available may also be displayed to the user on that occasion.

At step S50-C a user input is received which is directed to select one of the candidate finding types FT as the applicable finding type FT. According to some implementations, this information may be fed back to the actual prediction process in order to improve the workflow. For instance, the user selection may be provided to the prediction function PF for further adaptation / further training.

Finally, at step S50-D, the finding type FT thus selected by the user is included in the workflow as the finding type FT provided in step S50.

Figure 5 depicts optional method steps illustrating which kinds of information may be considered upon predicting the finding type FT. Thereby, the different approaches may be used individually or in arbitrary combinations in the step of predicting S50. Further, one or more of the steps may be implemented in the prediction function PF in the sense of the prediction function PF being configured to carry out one or more of these steps. The order of the steps does not necessarily correspond to the numbering of the steps but may also vary between different embodiments of the present invention. Further, individual steps or a sequence of steps may be repeated.

As mentioned, predicting the finding type FT may be performed by an appropriately configured prediction function PF. Accordingly, step S50 may comprise the optional sub-step S51 of providing the prediction function PF. For instance, the prediction function PF may be provided by holding it available in a memory of the processing unit, for instance, as executable computer code. In optional sub-step S52, the available input data, that is the user input INP, the representation R, the medical image data set MIDS, the supplementary information, the reference medical images, the displaying settings, and/or the imaging parameters, may be input into the prediction function PF which may then output the finding type FT (or a set of candidate finding types FT).

To improve the basis for the prediction of the finding type FT, supplementary information available for the patient may be considered. In optional sub-step S53 such supplementary medical information is obtained for the patient. To this end, the healthcare information system 40 may be queried for additional information available for the patient. To this end, a suitable search query may be formulated on the basis of an electronic identifier identifying the patient in the system. For instance, such identifier may be embodied by the patient name or a unique patient ID.

That followed, the supplementary information may be used to further narrow down potential finding types FT coming into question for a medical finding MF. For instance, if a prior report already indicated a certain finding type FT for the patient and a given organ, there is an increased likelihood that the same type of medical finding FT will still be present in the medical image data set MIDS.

Another option to further pinpoint the finding type FT is to systematically analyze which finding types FT have been reported for similar cases. To this end, the processing may rely on a set of reference medical images each depicting one or more medical findings MF with verified finding type FT. For instance, the finding type FT may have been verified by expert annotation or due to a confirmation in a previous reporting workflow. In particular, the reference medical images may stem from reference patients other than the patient. According to some examples, the reference medical images may be held available in a similar case database which may be part of the healthcare information system or the processing system 20.

To exploit the knowledge comprised in the reference cases, the method may comprise an optional sub-step S54. This optional sub-step S54 is directed to identify, from a plurality of reference medical images, at least one similar medical image based on degrees of similarity between the representation and individual reference medical images. As there is a certain likelihood that similar medical images also indicate similar finding types FT, the prediction of finding types FT can be improved for the case at hand. Specifically, the prediction of step S50 may then be based on at least one verified finding type FT of the identified similar medical images .

At step S55, a location information may be derived from the user input INP. Specifically, an anatomical location may be determined based on the user input INP. Thereby, the anatomical location may indicate the anatomy or anatomical coordinates the user input INP is directed to. For instance, a spot in the representation R may be identified based on the user input INP, and a relation of the spot to the medical image data set MIDS and/or the anatomy of the patient may be derived. For instance, the spot may be given in pixels of the representation R and the relation may be a relation to generalized coordinates and/or anatomic structures such as organs, anatomic landmarks, or atlases. Accordingly, an anatomical location thus derived may comprise an indication of an organ or organ part the user input INP is directed to or a relation of the user input to one or more anatomical landmarks. Furthermore, already a slice within the medical image data set MIDS, the representation R corresponds to can be used as anatomic location since already this piece of information indicates in what anatomy the user is interested in.

That followed, the location information may be used to further narrow down potential finding types FT coming into question for a medical finding MF. For instance, if the location information indicates a certain anatomy or organ, there is an increased likelihood that those finding types FT will have to be considered which are applicable for the certain anatomy or organ. In addition, also the supplementary medical information may be filtered based on the anatomical location in order to identify those pieces of information relating to the anatomical location and likely bearing an increased relevance for identifying the finding type FT.

As yet another source of information for narrowing down the potential finding types FT, the displaying settings currently applied by the user may be considered at step S56. Typically, displaying settings such as the lookup table, intensity, contrast, brightness or other settings are specific for a certain diagnostic task. Accordingly, exploiting the displaying settings may provide hints regarding the finding types FT coming into question.

Taking CT image data as an example, the representations are often intensity filtered by the user upon reading a study. Each voxel of a CT image data set usually has an intensity value that represents the attenuation of X-rays at the corresponding position in the volume as determined from the CT measurements (commonly measured in Hounsfield or HU units). Due to this relation, a kind of segmentation can be performed based solely on a thresholding of the voxel intensity values. For example, all voxels having intensity values in a particular range may be considered to represent bone. The corresponding range is called the "bone window". If such a window is detected, the finding type FT "fracture" may be likely. Other windows may relate to carve out soft tissue or lung parts. Here, the finding type FT "lung nodule" will have to be favored over the finding type FT "fracture".

Yet, further examples for displaying settings providing insights into possible finding types FT are auto-viewing settings. Auto-viewing settings often relate to a setting for a particular diagnostic task or a particular organ. The auto-viewing setting may be provided by the user interface for selection by the user. Since the auto-viewing settings are specific to a certain diagnostic task or anatomy, their knowledge may provide insights into the likely finding types FT. If, for instance, an auto-viewing setting for the liver is selected, finding types FT applicable for the liver become more likely.

To provide another example, also the viewing plane selected may provide further insights as to which finding type FT is pertinent. Typically, the viewing plane may be selected from an axial or sagittal or coronal viewing plane. In the diagnostic workflow, the viewing direction is an indication for the diagnostic question the user is looking at. Accordingly, the knowledge of the viewing plane can help to narrow down the finding types FT.

According to some examples, the display settings may also be considered in step S55 in the sense that the anatomic location may be derived additionally based on the displaying settings.

In medical imaging already the circumstances the acquisition process may provide insights into the underlying diagnostic questions. Accordingly, corresponding imaging parameters may be obtained and evaluated at step S57 in order to predict the finding types FT. In general, imaging parameters may relate to settings used during acquisition and/or pre- or post-processing of the medical image data set MIDS.

Specifically, imaging parameters may comprise such diverse information as a patient position during the image acquisition process, an image acquisition protocol used for acquiring raw data based on which the medical image data is generated, and/or an image reconstruction protocol used for generating the medical image data set MIDS based on raw data and so forth.

To provide an example, if the brain of the patient has been scanned, finding types FT of the brain will be considered in step S50 rather than finding types FT of the chest. Similarly, imaging protocols applicable for a certain disease may also indicate the presence of corresponding finding types FT.

Imaging parameters may be obtained as supplementary information or directly from the medical image data set MIDS where they may be stored as metadata.

To further improve the prediction of the finding types FT, the finding types FT generally coming into question may be categorized at step S58.

Here, each finding type FT in of all of the available finding types FT may be associated with at least one of a plurality of predetermined categories. With that, the finding types FT may be grouped into lists or buckets according to these categories. Then, in the step of predicting, only such finding types FT are considered the category of which matches a category indicated by the available input data such as the supplementary information, the displaying settings and/or the imaging parameters.

Associating finding types FT with overarching categories may bring about the advantage that the finding types FT may be efficiently preselected and grouped into lists according to their categories. According to some examples, the categories may comprise organs, suspected diagnosis, differential diagnosis, anatomic locations, stages of the patient in a disease trajectory and so forth. Another word for category may be diagnostic use case.

The association of the one or more categories may be performed using an appropriate ontology which maps a particular finding type FT to a superordinate category. In that sense, the method may further comprise the steps of defining a plurality of categories and associating each of the potential finding types FT to one or more of the plurality of categories.

Accordingly, step S58 may comprise determining an applicable category for the medical image data set MIDS based on one or more of: the medical image data set, the supplementary medical information, the anatomical location, the display settings, the imaging parameters, and/or the at least one similar image, and selecting the finding type FT by comparing the applicable category with the categories associated with the available finding types FT.

Figure 6 sets out an exemplary embodiment how the identification of similar medical images of step S54 may be put into practice. Figure 6 provides several examples in this regard but is not to be considered as limiting the scope of step S54 as it is not meant to exclude further possibilities how a similarity between image data can be determined. For instance, as an alternative to an explicit extraction of image descriptors and their ensuing comparison, a Fourier-based analysis scheme may be implemented (where individual images or image regions would be transformed into the Fourier space and a similarity would be calculated by applying mathematical convolution functions).

At step S54-A, an image descriptor is generated from the representation R. The image descriptor may comprise the representative or characterizing features of the representation R in the form of a feature vector. Since the representation may generally also comprise or be associated with non-image data, the image descriptor may likewise be based on image feature signatures and non-image features. Image feature signatures may be generated by image analysis methods comprising the identification, analysis and/or measurement of objects, local and or global structures and/or textures present in any image data comprised in the representation R. The generated image feature signatures may comprise an anatomical feature and/or structure, like, e.g., the presence of a landmark or the size of an organ or a structure, texture and/or density of an identified tissue or organ and so forth. The image feature signatures may likewise comprise a parameter characterizing a color and/or grey scale scheme or contrast characteristics or local gray scale gradients present in the analyzed image. The image feature signatures preferably comprise not only one but numerous features which as a sum characterize the analyzed image. The non-image features extracted from the non-image data comprised in the representation R may comprise meta-data associated to the image data. Further, they may relate to data independent form the image data providing further context information with regard to the target patient, such as features extracted from the electronic health record, laboratory data, and the like.

At step S54-B corresponding image descriptors are extracted from the reference medical images. The image descriptors of the reference medical images may be generated in the same way as the image descriptor(s) for the representation R. According to some examples, the image descriptors of the reference medical images already have been generated in advance and are stored together with the reference medical images, e.g., in the medical information system 40.

At step S54-C the image descriptor extracted from the representation R is compared to the image descriptors extracted from the reference medical images. According to some examples, the comparison of step S54-C may comprise determining a similarity or distance metric representing a similarity between the image descriptor of the representation R and a respective one of the image descriptors extracted from the reference medical images. In some examples, the similarity metric may be a distance in vector space between the image descriptor of the representation R and the respective one of the image descriptors of the reference medical images. For example, the distance may be the Euclidean distance between the two points in vector space that the respective image descriptors represent. In some other examples, the similarity metric may be based on the L1 norm of the respective image descriptors. In some further examples, other similarity metrics may be used, such as a cosine similarity between the data descriptors. For each reference medical image taken into account, the similarity metric may represent how similar the reference medical image is to the representation R. In other words, the similarity metric expresses (quantifies) a degree of similarity between the representation R and a respective reference medical image. The similarities determined in step S54-C may be used to select the image or those images of the reference medical images having the greatest similarity to the image data in the representation R. According to some examples a plurality of similar images may be identified on that basis.

According to some implementations, the aforementioned steps S54, S54-A, S54-B, S54-C may be carried out by an appropriately configured prediction function PF. It is to be understood, however, that the steps may also be carried out by a separate image analysis function.

Figure 7 depicts a schematic representation of the prediction function PF according to an embodiment. The prediction function PF according to this embodiment comprises a trained or machine-learned component FEX, CLA which is trained to predict a finding type FT based on input data IN-D comprising at least a user input INP directed to identify a medical finding MF in a representation R. It is to be understood however, that the functionalities of the prediction function PF may also be achieved without the usage of trained functions. For instance, one or more aspects described in the following may also be realized by one or more hard coded rules.

Generally, such machine-learned or trained functions FEX, CAS may relate to intelligent agents or classifiers suited for classifying data according to a learned task. This definition comprises, but is not limited to, data mining tools and techniques such as Support Vector Machines, decision trees, naive Bayes or (convolutional) neural networks NN. Specifically, according to an implementation, the prediction function PF may comprise one or more convolutional neural networks NN. In an embodiment, the arrangement of the prediction function PF comprises a fully convolutional neural network. Alternative network arrangements may be used, for example, a 3D Very Deep Convolutional Network (3D-VGGNet).

A convolutional neural network is defined in the form of a plurality of sequential layers. A first group of neural network layers FEX may be applied to extract features from the input data IN-D. The thus extracted features may be fed as input values to a second group of network layers, also known as classifiers CLA, which serve to further assign objects and/or characteristics to at least one of the extracted features present in the input data IN-D. Accordingly, the trained function may be seen as comprising at least an encoder branch FEX and a decoder branch CLA. According to some examples, the trained function may include multiple encoder branches FEX for different kinds of input data IN-D such as medical images R, prior reports, display setting, imaging parameters and the like. The decoder branch CLA may be configured to processes a merged latent descriptor data structure that is obtained from aggregating features obtained from the encoder branches. Specifically, the decoder branch CLA may map the aggregated feature into a (learned) feature space correlating distinct feature combinations with corresponding finding types FT. In particular, at least one of the encoder branches may be configured to extract a location information from the available input data IN-D.

Various types of layers may be used in the neural networks NN of the encoder FEX and decoder CLA branches, such as convolu-tional, pooling (e.g., max-pooling or average-pooling), up-sampling, deconvolutional, fully connected, or other types of layers. Convolutional layers convolve the input and pass its result to the next layer by moving an image filter kernel over the input. Pooling layers reduce the dimensions of the data by combining the outputs of node clusters at one layer into a single node in the next layer, thereby streamlining the underlying computation. Up-sampling and deconvolution layers reverse the actions of convolution and pooling layer in terms of the abstraction level. A fully connected layer connects every node in one layer to every node in another layer, so that essentially every feature gets a "vote". According to an implementation, skip connections may by used, so that layers may also output to other layers than the sequentially next layer introducing one or more residual blocks or layers. Such configuration is also referred to as ResNet. Using residual blocks results in the ability to train much deeper networks as this alleviates the vanishing gradient problem known from very deep neural networks.

Generally, the prediction function PF of this embodiment learns by adapting weights or weighting parameters of individual layers and nodes based on training data. Rather than pre-programming potential signs of finding types FT, the architecture of the prediction function PF may be defined to learn these patterns at different levels of abstraction based on input data IN-D. The prediction function PF may preferably be trained using a method according to supervised learning. Well established is the backpropagation method, which may be applied for embodiments of the present invention. During training, the prediction function PF is applied to training input values to produce corresponding output values the target values of which are known. The difference between produced and target output values (e.g., in the form of the mean squared error (MSE) of the difference between produced and target values) may be used to introduce a cost or loss function as a measure of how good or bad trained function TF performs. The goal of the training is to find a (local) minimum of the loss function by iteratively adjusting the weights of prediction function PF so that the prediction function PF is finally enabled to generate acceptable results across a (sufficiently) large cohort of training data. This optimization problem can be carried out using stochastic gradient descent or other approaches known in the art.

In principle, a prediction function PF comprising one or more encoder branches and a decoder branch can be trained by adapting either the encoding (i.e., the extraction of features) or the decoding (i.e., the classification according to finding types FT) or both. For instance, the one or more encoder branches can be adapted such that particularly meaningful feature sets are extracted. Further, the decoder branch may be trained such that an appropriate classification scheme is learned and/or applied.

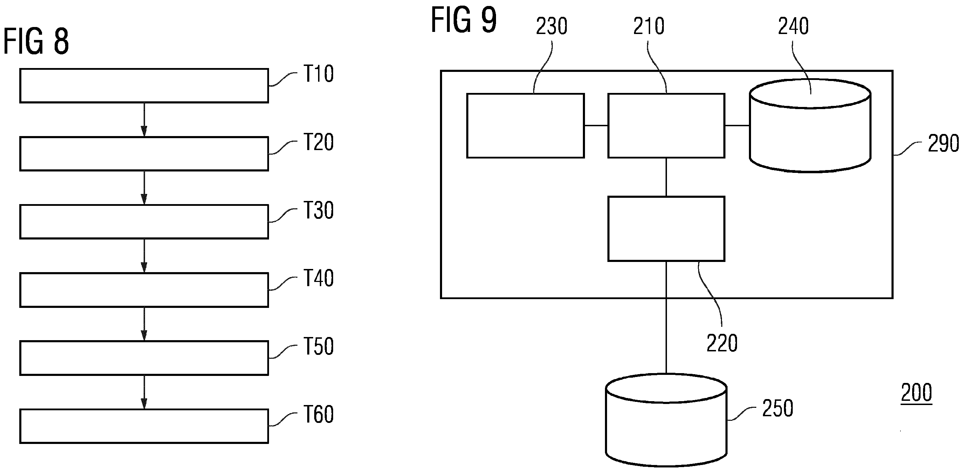

Figure 8 depicts a method for providing a prediction function PF to predict a finding type FT based on input data IN-D. Thereby, the input data IN-D comprises at least on user input INP indicating a medical finding MF in a medical image data set MIDS. The method comprises several steps. The order of the steps does not necessarily correspond to the numbering of the steps but may also vary between different embodiments of the present invention.

A first step T10 is directed to provide a plurality of training data sets. The training data sets respectively comprise a training input data set and a corresponding verified output data set. The training input data sets are preferably of the same type as the input data IN-D to be processed by the deployed and readily trained prediction function PF. Accordingly, the training input data sets each likewise comprise at least a user input INP indicating a medical finding in a medical image. Further, the training input data sets may comprise the medical image, supplementary information, imaging parameters, displaying settings, and the like as described before. The verified output data sets comprise verified finding types FT indicated by the corresponding training input data sets.

Next, at step T20, a training data set is provided to the (not readily trained) prediction function PF.

Based on the training input data of the training data set, the prediction function PF will determine a training finding type according to the learned task in step T30. In particular, the training finding type may be based on a user input INP and corresponding image data.

The performance of the prediction function PF (i.e., the quality of the predicted finding type) is evaluated in subsequent step T40 based on a comparison of the verified finding type FT and the training finding type.

The comparison is used as a loss function to adjust weights of the prediction function PF at step T50.

At step T60 the steps of obtaining a training finding type (step T30) and comparing the result to the verified finding type (step T40) are repeated with paired sets of training input data sets and output data sets until the prediction function PF is able to generate results that are acceptable (i.e., until a local minimum of the loss function is reached). Once all pairs have been used, pairs are randomly shuffled for the next pass.

Figure 9 illustrates an embodiment of a system 200 for training a prediction function PF. The system comprises a processor 210, an interface 220, a memory 230, a storage 240, and a database 250. Processor 210, interface 220, memory 230 and storage 240 may be embodied by a computer 290. Processor 210 controls the overall operation of the computer 200 by executing computer program instructions which define such operation. The computer program instructions may be stored in memory 230 or in storage 240 and loaded into memory 230 when execution of the computer program instructions is desired. Storage 240 may be a local storage as a component of the system 200, or a remote storage accessible over a network, such as a component of a server or cloud system. The method steps illustrated in Figure 8 may be defined by the computer program instructions stored in memory 230 and/or storage 240 and controlled by processor 210 executing the computer program instructions.

Database 250 is a storage device such a cloud or local storage serving as an archive for the training data sets comprising as introduced above. Database 250 may be connected to computer 290 for receipt of one or more medical images. It is also possible to implement database 250 and computer 290 as a single device. It is further possible that database 250 and computer 290 communicate wirelessly or with wired connection through a network. Interface 220 is configured to interact with database 250.

Wherever meaningful, individual embodiments or their individual aspects and features can be combined or exchanged with one another without limiting or widening the scope of the present invention. Advantages which are described with respect to one embodiment of the present invention are, wherever applicable, also advantageous to other embodiments of the present invention.

## Claims

1. Computer-implemented method for providing a template data structure (TDS) for a medical report (MER) comprising the following steps:
- receiving (S10) a medical image data set (MIDS) of a patient,
- generating (S20) a representation (R) of the medical image data set (MIDS) for displaying to a user via a user interface (10),
- providing (S30) the representation (R) for displaying to the user via the user interface (10),
- receiving (S40) a user input (INP) via the user interface (10) directed to a medical finding (MF) visible in the representation (R),
- predicting (S50) a finding type (FT) of the medical finding (MF) based on the user input (INP),
- performing (S60) a lookup operation in a database (RD) to identify a template data structure (TDS) for a medical report (MER) corresponding to the predicted finding type (FT),
- providing (S70) the identified template data structure (TDS) .

2. Method according to claim 1, further comprising
- providing (S51) a prediction function (PF) configured to predict a finding type (FT) of medical findings (MF) based on at least user inputs (INP) made with respect to representations (R) of medical image data sets(MIDS),
- wherein:
- the step of predicting (S50) comprises applying (S52) the prediction function (PF) to the user input (INP), and
- optionally, the prediction function (PF) comprises a machine-learned function (FEX, CLA) trained to predict a finding type (FT) based on at least user inputs(INP) made with respect to representations (R) of medical image data sets (MIDS).

3. Method according to claim 2, wherein
- the machine-learned function (FEX, CLA) has been trained based on medical image data sets (MIDS) respectively depicting a body part of a patient and comprising at least one medical finding (MF) with a verified finding type (FT).

4. Method according to any one of the preceding claims, further comprising:
- obtaining (S53) supplementary medical information of the patient,
- wherein the step of predicting (S50) is further based on the supplementary medical information.

5. Method according to claim 4, wherein
- the supplementary medical information comprises one or more of the following elements:
- a prior medical report of the patient, and/or
- a-priori knowledge of at least one type of medical problem the patient is suspected to have, and/or
- an indication of a diagnostic task to be performed based on the medical image data set (MIDS) for the patient, and/or
- a medical guideline applicable for the patient, and/or
- an electronic health record of the patient.

6. Method according to any one of the preceding claims, wherein
- the method comprises identifying (S54), from a plurality of reference medical images of reference patients different than the patient, at least one similar medical image based on degrees of similarity between the representation (R) and individual reference medical images,
- each reference medical image comprises one or more findings with a verified finding type, and
- the step of predicting (S50) is further based on at least one verified finding type of the identified at least one similar medical image.

7. Method according to claim 6, wherein
- the step of identifying (S54) the at least one similar medical image comprises:
- extracting (S54-A) an image descriptor from the representation (R);
- respectively extracting (S54-B) a corresponding image descriptor from each one of the plurality of reference medical images, and
- determining (S54-C), for each one of the plurality of reference medical images, a similarity metric indicative of the degree of similarity between the image descriptor of the representation (R) and the respective image descriptor of the respective reference medical image.

8. Method according to any one of the preceding claims, wherein
- the method further comprises determining (S55) an anatomical location the user input (INP) is directed to based on the user input (INP) and the representation (R) and/or the medical image data set (MIDS), and
- the step of predicting (S50) the finding type (FT) is further based on the anatomical location.

9. Method according to any one of the preceding claims, wherein
- the method further comprises obtaining (S56) one or more displaying settings applied by the user for the representation (R) upon displaying the representation (R), and
- the step of predicting (S50) the finding type (FT) is further based on the displaying settings.

10. Method according to any one of the preceding claims, wherein
- the method further comprises obtaining (S57) at least one imaging parameter of the medical image data set (MIDS), the imaging parameter relating to settings used during acquisition and/or pre-processing of the medical image data set (MIDS), and
- the step of predicting (S50) is further based on the imaging parameter.

11. Method according to any one of the preceding claims, wherein
- the user input (INP) is directed to generate a measurement of an image feature depicted in the representation (R), and
- the step of predicting (S50) the finding type (FT) is further based on the measurement.

12. Method according to any one of the preceding claims, wherein
- the step of predicting (S50) comprises:
- predicting (S50-A) a plurality of likely finding types for selection by the user,
- providing (S50-B) the plurality of likely finding types to the user,
- receiving (S50-C) a user input directed to select one of the likely finding types,
- providing (S50-D) the finding type (FT) based on the user selection.

13. System (1) for providing a template data structure (TDS) for a medical report (MER), the system (1) comprising an interface unit (10), a computing unit (20), and a database (RD) ,
- the database (RD) being configured to store a plurality of report template structures (TDS) for medical reports (MER), each report template structure (TDS) corresponding to a distinct finding type (FT) of a medical finding(MF),
- the interface unit (10) being configured to:
- receive (S10) a medical image data set (MIDS) of a patient,
- forward (S30) a representation (R) of the medical image data set (MIDS) for displaying to a user,
- receive (S40) a user input (INP) from the user directed to indicate a medical finding (MF) in the representation (R), and
- provide (S70) the template data structure(TDS) to the user, and
- the computing unit (20) being configured to
- generating (S20) the representation (R) of the medical image data set (MIDS) for displaying to the user,
- predict (S50) a finding type (FT) of the medical finding (MF) based on the user input (INP), and
- perform (S60) a lookup operation in the database (RD) to identify a template data structure (TDS) corresponding to the predicted finding type (FT).

14. Computer program product comprising program elements which induce a computing unit (20) of a system (1) for providing a template data structure (TDS) for a medical report (MER) to perform steps of the method according to any of claims 1 to 12 when the program elements are loaded into a memory of the computing unit (20).

15. Computer-readable medium on which program elements are stored that are readable and executable by a computing unit (20) of a system (1) for providing a template data structure (TDS) for a medical report (MER) to perform steps of the method according to any of claims 1 to 12 when the program elements are executed by the computing unit (20).
